(19) Europäisches Patentamt
European Patent Office
Office européen des brevets

(11) Publication number : **0 382 935 B1**

(12) # EUROPEAN PATENT SPECIFICATION

(45) Date of publication of patent specification : **22.07.92 Bulletin 92/30**

(51) Int. Cl.$^5$ : **C07D 409/04,** A61K 31/415, A61K 31/38

(21) Application number : **89123377.7**

(22) Date of filing : **18.12.89**

(54) **2-(1H-imidazol-4(5)-yl)-3,4-dihydro-2H-1-benzothiopyran.**

(30) Priority : **15.02.89 GB 8903437**

(43) Date of publication of application : **22.08.90 Bulletin 90/34**

(45) Publication of the grant of the patent : **22.07.92 Bulletin 92/30**

(84) Designated Contracting States : **DE GB IT**

(56) References cited :
**EP-A- 0 183 492**
**EP-A- 0 289 066**

(73) Proprietor : **FARMITALIA CARLO ERBA S.r.L.**
**Via Carlo Imbonati 24**
**I-20159 Milano (IT)**

(72) Inventor : **Carganico, Germano**
**via Domodossola 7**
**Milano (IT)**
Inventor : **Cozzi, Paolo**
**via Zanella 48/5**
**Milano (IT)**
Inventor : **Vaghi, Fabrizio**
**via Cavour 3**
**Villaguardia (Como) (IT)**
Inventor : **Salvati, Patricia**
**via Valera 16**
**Arese (Milano) (IT)**

(74) Representative : **Ferrario, Vittorino**
**c/o Farmitalia Carlo Erba S.r.l. Via Carlo Imbonati 24**
**I-20159 Milano (IT)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Jouve, 18, rue Saint-Denis, 75001 PARIS

## Description

The present invention relates to 2-(1H-imidazol-4(5)-yl)-3,4-dihydro-benzothiopyran, to a process for its preparation and to pharmaceutical compositions containing it. EP-A-0289066 relates to 1H-imidazoles, substituted in particular at the 1-position by a condensed ring system, having herbicidal properties.

EP-A-0183492 discloses 4(5)-substituted imidazole derivatives having selective $\alpha_2$-antagonistic activity.

The invention provides the compound 2-(1H-imidazol-4(5)-yl)-3,4-dihydro-2H-1-benzothiopyran having the following formula (I)

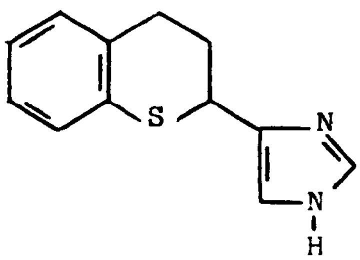

(I)

The present invention includes all the possible isomers, e.g. diastereoisomers and enantiomers, of the compounds of formula (I) and their mixtures.

Pharmaceutically acceptable salts of the compound of formula (I) include acid addition salts, with inorganic, e.g. nitric, hydrochloric, hydrobromic, sulphuric, perchloric and phosphoric acids, or organic e.g. acetic, propionic, glycolic, lactic, oxalic, malonic, malic, maleic, tartaric, citric, benzoic, cinnamic, mandelic, fumaric, methanesulfonic and salicyclic acids, and salts with inorganic, e.g. alkali metal, especially sodium or potassium, bases or alkaline earth metal, especially calcium or magnesium, bases, or with organic bases, e.g. alkylamines, preferably triethylamine.

The compound of the invention, and the salts thereof, can be prepared by a process comprising reacting a compound of formula (II)

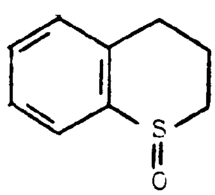

(II)

with a reactive derivative of imidazole, and, if desired, converting a compound of formula (I) into a salt thereof, and/or, if desired, converting a salt into a free compound, and/or, if desired, resolving a mixture of isomers of compounds of formula (I) into the single isomers. A reactive derivative of imidazole is for example a N-(trialkylsilyl)-imidazole, preferably a N-($C_1$-$C_6$-trialkyl-silyl)-imidazole, more preferably N-(trimethyl-silyl)-imidazole or N-(tert. butyl-dimethyl-silyl)-imidazole.

The reaction between a compound of formula (II) and a reactive derivative of imidazole as mentioned above is preferably carried out at a temperature ranging from 120°C to 200°C more preferably from 150°C to 175°C, and, if necessary in the presence of a suitable aprotic organic solvent, preferably dimethylacetamide or dimethylformamide. The reaction time may range from 2 to 16 hours; preferably from 5 to 10 hours.

Alternatively the process may be also carried out by treating a compound of formula (II), as defined above, with imidazole and a suitable silylating agent, preferably bis-(trimethylsilyl)-urea or hexamethyldisilazane, so as to form the N-(trialkyl-silyl)-imidazole in situ.

The compound of formula (II) is a known compound, or it may be prepared by known methods from known compounds, e.g. by oxidizing the corresponding 3,4-dihydro-2H-1-benzothiopyran of formula (III)

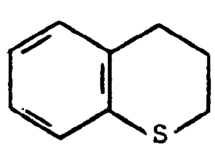

(III)

with a suitable oxidizing agent, preferably by treating with hydrogen peroxide in acetic acid at a temperature ranging from -20°C to +50°C, or with sodium metaperiodate or with tert. butyl hypochlorite, in a suitable solvent, e.g., a $C_1$-$C_4$ alkyl alcohol, in particular methanol, or water or mixtures thereof, preferably methanol water, at a temperature ranging from -20°C to +50°C.

The optional salification of a compound of formula (I) as well as the conversion of a salt into the free compound and the separation of a mixture of isomers into the single isomers may be carried out by conventional methods.

The compound of the invention has been found active in regulating noradrenaline (NA) balance, as is proven by its affinity to $\alpha_2$-adrenergic receptors, e.g. in the yohimbine binding test (T.M. Lavin, B.B. Hoffman and R.J. Lefkowitz, Molecular Pharmacology, 20, 28 (1981)) for $\alpha_2$-receptor affinity.

The activity of the compound has been evaluated, e.g. in the isolated organ, in particular in the rat vas deferens test for the $\alpha_2$ adrenergic activity (M.D. Geoffrey, Eur. J.Pharmacol. 42, 123 (1977)).

For the $\alpha_2$ adrenergic agonistic activity in vivo the hypotensive effect in the conscious chronically cannulated S.H.R rats (Leonard I., Kleinman E., Edward P., Radford Jr. and Torelli G., Am.J.Physiol., 208 (3): 578-584 (1965)) was evaluated.

The compound 2-(1H-imidazol-4-(5)-yl)-3,4-dihydro-2H-1-benzothiopyran showed a marked affinity for $\alpha_2$ adrenergic receptors, with an $IC_{50} = 3 \times 10^{-9}$M ($IC_{50}$ for $\alpha_1$ receptors = $8.5 \times 10^{-7}$M, $\alpha_2/\alpha_1$ ratio = 283).

The same compound acted as an $\alpha_2$-adrenoreceptor agonist on the vas deferens, with an $IC_{50} = 1.2 \times 10^{-8}$M, and had a valuable hypotensive effect paralleled by a decrease in heart rate in the conscious, chronically cannulated spontaneously hypertensive rats (SHR) at a dose of 1 mg/kp p.o.

Therefore the compound of the invention can be used in the alleviation, treatment or amelioration of any of numerous indications which are sensitive to changes in the noradrenaline balance, for example thrombosis, constipation, paralytic ileus, senile dementia, alcoholism, memory impairment, phobic anxiety; it can find also use as antihypertensive agent.

The toxicity of the compound of the invention is negligible, therefore it can be safety used in therapy.

Nine hours food deprived mice and rats were treated orally with single administration of increasing doses, then housed and normally fed. The orientative acute toxicity ($LD_{50}$) was assessed on the seventh day after the treatment.

The compound of the invention can be administered in a variety of dosage forms, e.g., orally, in the form of tablets, capsules, sugar or film coated tablets, liquid solutions or suspensions; rectally, in the form of suppositories; or parenterally, e.g. intramuscularly or by intravenous injection or infusion.

In emergency situations the preferred route of administration is intravenous. The suitable dosage depends, as usual, upon the route and purpose of administration, the form in which the compound is administered and the age, weight and conditions of the subject involved. The compound of the invention is preferably administered at the usual therapeutic doses of the known drugs having similar pharmacological activity; for instance it can be administered for the treatment of hypertension in adult humans in a dosage ranging from 5 to 250 mg/day, preferably from 10 to 100 mg/day.

The invention includes pharmaceutical compositions comprising a compound of the invention in association with a pharmaceutically acceptable excipient (which can be a carrier or diluent).

The pharmaceutical compositions containing the compound of the invention are usually prepared following conventional methods and are administered in a pharmaceutically suitable form.

For example, the solid oral forms may contain, together with the active compound, diluents, e.g. lactose, dextrose, saccharose, cellulose, corn starch or potato starch; lubricants, e.g. silica, talc, stearic acid, magnesium or calcium sterate, and/or polyethylene glycols; binding agents, e.g. starches, arabic gums, gelatin, methylcellulose, carboxymethylcellulose or polyvinyl pyrrolidone; disaggregating agents, e.g. a starch, alginic acid, alginates or sodium starch glycolate; effervescing mixtures, dyestuffs; sweeteners; wetting agents, such as lecithin, polysorbates, laurylsulphates, and in general, non-toxic and pharmacologically inactive substances used in pharmaceutical formulations. Said pharmaceutical preparations may be manufactured in known manner, for example, by means of mixing, granulating, tabletting, sugar-coating or film-coating processes.

The liquid dispersions for oral administration may be, e.g., syrups, emulsions, and suspensions. The syrups may contain as carrier, for example, saccharose or saccharose with glycerine and/or mannitol and/or sorbitol.

The suspensions and the emulsions may contain as carrier, for example, a natural gum, agar, sodium alginate, pectin, methylcellulose, carboxymethylcellulose, or polyvinyl alcohol. The suspensions or solutions for intramuscular injections may contain,together with the active compound, a pharmaceutically acceptable carrier, e.g. sterile water, olive oil, ethyl oleate, glycols, e.g., propylene glycol, and if desired, a suitable amount of lidocaine hydrochloride.

The solutions for intravenous injections or infusions may contain as carrier, for example sterile water or

preferably they may be in the form of sterile, aqueous, isotonic saline solutions.

The suppositories may contain, together with the active compound, a pharmaceutically acceptable carrier, e.g. cocoabutter, polyethylene glycol, a polyoxyethylene sorbitan fatty acid ester surfactant or lecithin.

The N.M.R spectra were measured in a solution of dimethyl sulphoxide-$d_6$ or of $CDCl_3$, using a 90 M-hertz Bruker HFX apparatus.

The $R_f$ values were determined by thin layer chromatography on ready - to-use silica gel plates of 0.25 mm coating thickness.

Example 1

A mixture of 2 g of 3,4-dihydro-2H-1-benzothiopyran-1-oxide, 5 g of imidazole and 2.2 g of hexamethyldisilazane is heated at 150°C for 12 hours with vigorous stirring.

The mixture is cooled, poured into ice/water and extracted with ethyl acetate.

The solution is dried over $Na_2SO_4$ and the solvent is evaporated under vacuum.

The crude product is chromatographed on silica gel (eluant: ethyl acetate + 1% of conc. $NH_4OH$), giving 0.6 g of 2-(1H-imidazol-4(5)-yl)-3,4-dihydro-2H-1-benzothiopyran, m.p. 157-159°C.
Elemental analysis
Found: C 66.25; H 5.61; N 12.85; S 14.64
Calculated for $C_{12}H_{12}N_2S$ : C 66.63; H 5.59; N 12.95; S 14.82
T.L.C.: eluant ethyl acetate + 2% conc. $NH_4OH$ Rf = 0.27.
NMR (DMSO - $d_6$) δ ppm:
2.25 (2H, m, $CH_2\underline{CH_2}$ CH-S)
2.88 (2H, m, $CH_2$ $CH_2$ CH - S)
4.50 (1H , dd, S-CH-N)
7.00 (5H, m, phenyl + H-5 imidazole)
7.67 (1H, d, H-2 imidazole)

Example 2

2-(1H-imidazol-4(5)-yl)-3,4-dihydro-2H-1-benzothiopyran, dissolved in absolute ethanol, is treated with a stoichiometric amount of hydrogen chloride, to give 2-(1H-imidazol-4(5)-yl)-3,4-dihydro-2H-1-benzothiopyran hydrochloride.

Example 3

Tablets, each weighing 150 mg and containing 50 mg of the active substance can be manufactured as follows:
Compositions (for 10,000 tablets)
2-(1H-imidazol-4(5)-yl)-3,4-dihydro-2H-1-benzothiopyran 500 g

| | |
|---|---|
| Lactose | 710 g |
| Corn starch | 237.5 g |
| Talc powder | 37.5 g |
| Magnesium stearate | 15 g |

2-(1H-imidazol-4(5)-yl)-3,4-dihydro-2H-1-benzothiopyran, lactose and a half of the corn starch are mixed; the mixture is then forced through a sieve of 0.5 mm openings.
Corn starch (18 g) is suspended in warm water (180 ml).

The resulting paste is used to granulate the powder. The granules are dried, comminuted on a sieve of sieve size 1.4 mm, then the remaining quantity of starch, talc and magnesium stearate is added, carefully mixed, and processed into tablets using punches of 8 mm diameter.

**Claims**

1. The compound 2-(1H-imidazol-4(5)-yl)-3,4-dihydro-2H-1-benzothiopyran having the following formula (I)

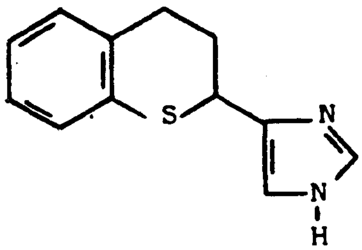

(I)

and the pharmaceutically acceptable salts thereof.

2. A process for the preparation of the compound of formula (I) and the salts thereof, according to Claim 1, comprising reacting a compound of formula (II).

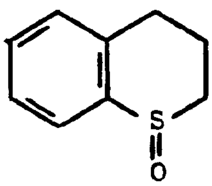

(II)

with a reactive derivative of imidazole, and, if desired, converting a compound of formula (I) into a salt thereof, and/or, if desired, converting a salt into a free compound, and/or, if desired, resolving a mixture of isomers of compounds of formula (I) into the single isomers.

3. A process according to claim 2 wherein the reactive derivative of imidazole in as N-(trialkyl silyl)-imidazole which is formed in situ, which comprises treating the compound of formula (II) as defined in claim 2 with imidazole and a silylating agent.

4. A pharmaceutical composition comprising a suitable carrier and/or diluent and, as an active principle, a compound of formula (I) according to Claim 1 or a pharmaceutically acceptable salt thereof.

5. A compound of formula (I) or salt thereof according to claim 1 for use in the alleviation, treatment or amelioration of a medical indication that is sensitive to change in the noradrenaline balance.

6. A compound or salt according to claim 5 wherein the indication is thrombosis, constipation, paralytic ileus, senile dementia, alcoholism, memory impairement or phobic anxiety.

7. A compound or salt according to claim 5 for use in the treatment of hypertension.

**Patentansprüche**

1. Die Verbindung 2-(1H-Imidazol-4(5)-yl)-3,5-dehydro-2H-1-benzothiopyran mit der nachstehenden Formel (I)

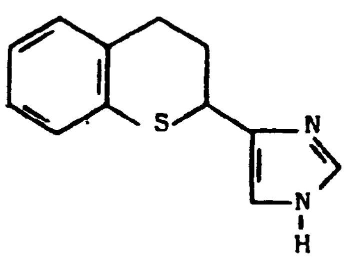

(I)

und die pharmazeutisch annehmbaren Salze derselben.

2. Verfahren zur Herstellung einer Verbindung der Formel (I) und der Salze derselben gemäss Anspruch 1, dadurch gekennzeichnet dass es die Reaktion einer Verbindung der Formel (II)

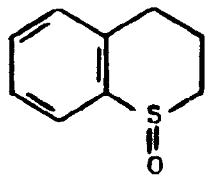

( II )

mit einem reaktionsfähigen Imidazolderivat und, wenn gewünscht, die Umwandlung einer Verbindung der Formel (I) in ein Salz derselben, und/oder, wenn gewünscht, die Umwandlung eines Salzes in eine freie Verbindung, und/oder, wenn gewünscht, die Auflösung eines Isomergemisches der Verbindungen der Formel (I) in die einzelnen Isomeren umfasst.

3. Verfahren gemäss Anspruch 2, worin das reaktionsfähige Imidazolderivat ein in situ gebildetes N-(Trialkylsilyl)-imidazol ist, dadurch gekennzeichnet, dass es die Bearbeitung der Verbindung der Formel (II) wie sie im Anspruch 2 definiert ist mit Imidazol und einem Silylationsagens umfasst.

4. pharmazeutisches Kombinationspräparat, dadurch gekennzeichnet, dass es einen angemessenen Träger und/oder Verdünner und, als Wirkstoff, eine Verbindung der Formel (I) gemäss Anspruch 1 oder ein pharmazeutisch annehmbares Salz derselben umfasst.

5. Verbindung der Formel (I) oder ein Salz derselben gemäss Anspruch 1, dadurch gekennzeichnet, dass sie zur Milderung, Behandlung und Besserung einer ärztlichen Indikation, die auf die Änderungen des Noradrenalingleichgewichts empfindlich ist, angewendet wird.

6. Eine Verbindung oder ein Salz gemäss Anspruch 5, dadurch gekennzeichnet, dass die Indikationen Thrombose, Konstipation, paralytischer Ileus, Dementia senilis, Alkoholismus, Gedächtnisschwund oder Angst bei Phobie lauten.

7. Eine Verbindung oder ein Salz gemäss Anspruch 5, dadurch gekennzeichnet dass sie zur Behandlung der Hypertonie angewendet wird.

## Revendications

1. Le composé 2-(1H-imidazol-4(5)-yl)-3,4-déhydro-2H-1-benzothiopyrane possédant la suivante formule (I)

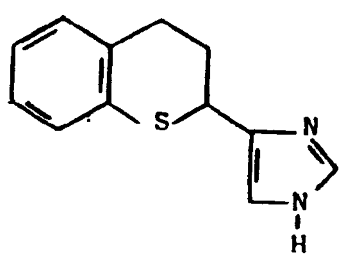

(I)

et les sels de ce dernier acceptables du point de vue pharmaceutique.

2. Procédé de fabrication du composé de la formule (I) et des sels de ce dernier selon la revendication 1, caractérisé par le fait qu'il comprend la réaction d'un composé représenté par la formule (II)

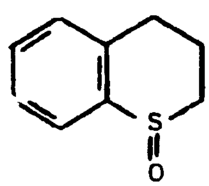

( II )

avec un réactif dérivé de l'imidazole et, si désiré, la conversion d'un composé de la formule (I) en l'un des sels de ce dernier, et/ou, si désiré, la conversion d'un sel en un composé libre, et/ou, si désiré, la résolution d'un mélange d'isomères des composés de la formule (I) dans les isomères singuliers.

3. procédé selon la revendication 2 où le réactif dérivé de l'imidazole est un N-(trialkyl-silyl)-imidazole formé in situ, caractérisé par le fait qu'il comprend le traitement du composé de la formule (II) tel que défini à la revendication 2 par imidazole et un agent de silylation.

4. Composition pharmaceutique, caractérisée par le fait qu'elle comprend un support et/ou diluant convenable et, comme substance active, un composé de la formule (I) selon la revendication 1 ou l'un des sels de ce dernier acceptables du point de vue pharmaceutique.

5. Composé de la formule (I) ou un des sels de ce dernier selon la revendication 1, caractérisé par le fait que l'on l'emploie pour alléger, traiter et améliorer une indication médicale sensible au changement de l'équilibre de la noradrénaline.

6. Composé ou sel selon la revendication 5, caractérisé par le fait que son indication est constitué par thrombose, constipation, iléon paralytique, démence sénile, alcoolisme, perte de mémoire ou anxiété phobique.

7. Composé ou sel selon la revendication 5, caractérisé par le fait que l'on l'emploie dans le traitement de l'hypertension.